# EUROPEAN PATENT SPECIFICATION

(11) **EP 2 338 487 B1**
(45) Date of publication and mention of the grant of the patent: **11.09.2013**
(21) Application number: 11157696.3
(22) Date of filing: 17.01.2007
(51) Int. Cl.: C07D 403/04, A61K 41/00, A61K 45/06, A61K 31/4164, C07D 401/02, C07D 471/00, A61K 49/00, A61K 9/00, A61P 35/00, A61N 5/00, A61K 31/41

(54) **Combination therapy with PARP inhibitors**
Kombinationstherapie mit PARP Inhibitoren
Thérapie combinée comportant des inhibiteurs de PARP

(30) Priority: 17.01.2006 US 759445 P; 07.06.2006 US 804112 P; 06.10.2006 US 850042 P; 12.10.2006 US 829261 P; 28.11.2006 US 867518 P
(43) Date of publication of application: 29.06.2011
(62) Divisional of application: 07716699.9
(73) Proprietor: Abbott Laboratories, Abbott Park, Illinois 60064-6008 (US)
(72) Inventor: Wernet, Wolfgang, 67434, Neustadt (DE); Penning, Thomas, D., Elmhurst, IL 60126 (US); Giranda, Vincent, L., Gurnee, IL 60031 (US)
(74) Representative: Modiano, Micaela Nadia

(56) References cited:
- WO-A-00/32579
- WO-A-97/04771
- WO-A-2006/052976
- WO-A-2006/110816
- JODI A MUSCAL ET AL: "Plasma and cerebrospinal fluid pharmacokinetics of ABT-888 after oral administration in non-human primates", CANCER CHEMOTHERAPY AND PHARMACOLOGY, SPRINGER, BERLIN, DE, vol. 65, no. 3, 13 June 2009 (2009-06-13), pages 419-425, XP019779203, ISSN: 1432-0843

## Description

### FIELD OF THE INVENTION

The invention relates to compositions comprising drugs having additive anti-cancer activity and methods of treatment using the combinations.

### Background

Poly(ADP-ribose)polymerase (PARP) or poly(ADP-ribose)synthase (PARS) has an essential role in facilitating DNA repair, controlling RNA transcription, mediating cell death, and regulating immune response. These actions make PARP inhibitors targets for a broad spectrum of disorders. PARP inhibitors have demonstrated efficacy in numerous models of disease, particularly in models of ischemia reperfusion injury, inflammatory disease, degenerative diseases, protection from adverse effects of cytoxic compounds, and the potentiation of cytotoxic cancer therapy. PARP has also been indicated in retroviral infection and thus inhibitors may have use in antiretroviral therapy, PARP inhibitors have been efficacious in preventing ischemia reperfusion injury in models of myocardial infarction, stroke, other neural trauma, organ transplantation, as well as reperfusion of the eye, kidney, gut and skeletal muscle. Inhibitors have been efficacious in inflammatory diseases such as arthritis, gout, inflammatory bowel disease, CNS inflammation such as MS and allergic encephalitis, sepsis, septic shock, hemmorhagic shock, pulmonary fibrosis, and uveitis. PARP inhibitors have also shown benefit in several models of degenerative disease including diabetes (as well as complications) and Parkinsons disease. PARP inhibitors can ameliorate the liver toxicity following acetominophen overdose, cardiac and kidney toxicities from doxorubicin and platinum based antineoplastic agents, as well as skin damage secondary to sulfur mustards. In various cancer models, PART inhibitors have been shown to potentiate radiation and chemotherapy by increasing apoptosis of cancer cells, limiting tumor growth, decreasing metastasis, and prolonging the survival of tumor-bearing animals.

### Summary of the Invention

The present invention describes a specific potent PARP inhibitor in combination with radiotherapy for use in treating CNS tumors.

### Detailed Description of the Drawings

Figure 1 shows data generated from the single and combined administration of the compound, 2-(N-propylpiperidin-4-yl) benzirnidazale-4-carbaxamide and radiotherapy.
Figure 2 shows data generated from the single and combined administration of A-861695 and TMZ in rats with murine melanoma.
Figure 3 shows data generated from the single and combined administration of A-861695 and TMZ in rats with orthotopic gliosarcoma.
Figure 4 shows data generated from the single and combined administration of A-861695 and carboplatin in the MX-1 breast carcinoma xenograft model in scid mice.
Figure 5 shows data generated from the single and combined administration A-861695 and cisplatin in the MX-1 breast carcinoma xenograft model in nude mice.
Figure 6 shows data generated from the single and combined administration valproic acid and radiotherapy.
Figure 7 shows the survival rate of mice with intra-cerebellar medulloblastoma xenographs after having been treated with TMZ and ABT-888 in combination and as single agents.
Figure 8 shows the survival rate of mice with intra-cerebellar medulloblastoma xenographs after having been treated with TMZ and ABT-888 in combination and as single agents.
Figure 9 shows results of administration of differing amounts of TMZ and ABT-888 combinations for HSB T-cell ALL
Figure 10 shows results of administration of differing amounts of TMZ and ABT-888 combinations for JM1 pre-B ALL.
Figure 11 shows results of administration of differing amounts of TMZ and ABT-888 combinations for P 115 primary AML cells.

### Detailed Description of the Invention

The present invention provides the administration of 2-[(2R)-2-methylpyrrolidin-2-yl]-1H-benzimidazole-4-carboxamide or a therapeutically acceptable salt thereof, in combination with radiotherapy for use in treating cancer or inhibiting tumor growth in a mammal, wherein the cancer is a CNS tumor.

In another embodiment, the present invention provides a method of inhibiting tumor growth in a mammal in recognized need of such treatment comprising administering to the mammal a therapeutically acceptable amount of 2-[(2R)-2-methylpyrrolidin-2-yl]-1H-benzimidazole-4-carboxamide, or a therapeutically acceptable salt thereof, and radiotherapy.

### Definitions

Proper valences are maintained for all moieties and combinations thereof of the compounds of this invention.

As used throughout this specification and the appended claims, the following terms have the following meanings:

The term "leukemia," as used herein means acute myleogenous leukemia, lymphocytic leukemia or chronic myleoid leukemia.

The term "A-861695," and the term "ABT-888" as used herein is the compound 2-[(2R)-2-methylpyrrolidin-2-yl]-1H-benzimidazole-4-carboxamide.

The term "ABT-472," as used herein means the compound 2-(N-propylpiperidin-4-yl) benzimidazole-4-carboxamide.

The term "alkenyl" as used herein, means a straight or branched chain hydrocarbon containing from 2 to 10 carbons and containing at least one carbon-carbon double bond formed by the removal of two hydrogens. Representative examples of alkenyl include, but are not limited to, ethenyl, 2-propenyl, 2-methyl-2-propenyl, 3-butenyl, 4-pentenyl, 5-hexenyl, 2-heptenyl, 2-methyl-1-heptenyl, and 3-decenyl.

The term "alkoxy" as used herein, means an alkyl group, as defined herein, appended to the parent molecular moiety through an oxygen atom, Representative examples of alkoxy include, but are not limited to, methoxy, ethoxy, propoxy, 2-propoxy, butoxy, tert-butoxy, pentyloxy, and hexyloxy.

The term "alkoxyalkyl" as used herein, means at least one alkoxy group, as defined herein, appended to the parent molecular moiety through an alkyl group, as defined herein. Representative examples of alkoxyalkyl include, but are not limited to, tert-butoxynnethyl, 2-ethoxyethyl, 2-methoxyethyl, and methoxymethyl.

The term "alkoxycarbonyl" as used herein, means an alkoxy group, as defined herein, appended to the parent molecular moiety through a carbonyl group, as defined herein. Representative examples of alkoxycarbonyl include, but are not limited to, methoxycarbonyl, ethoxycarbonyl, and tert-butoxycarbonyl.

The term "alkoxycarbonylalkyl" as used herein, means an alkoxycarbonyl group, as defined herein, appended to the parent molecular moiety through an alkyl group, as defined herein.

The term "alkyl" as used herein, means a straight or branched chain hydrocarbon containing from 1 to 10 carbon atoms. Representative examples of alkyl include, but are not limited to, methyl, ethyl, n-propyl, iso-propyl, n-butyl, sec-butyl, iso-butyl, tert-butyl, n-pentyl, isopentyl, neopentyl, n-hexyl, 3-methylhexyl, 2,2-dimethylpentyl, 2,3-dimethylpentyl, n-heptyl, n-octyl, n-nonyl, and n-decyl.

The term "alkylcarbonyl" as used herein, means an alkyl group, as defined herein, appended to the parent molecular moiety through a carbonyl group, as defined herein. Representative examples of alkylcarbonyl include, but are not limited to, acetyl, 1-oxopropyl, 2,2-dimethyl-1-oxopropyl, 1-oxobutyl, and 1-oxopentyl.

The term "alkylcarbonyloxy" as used herein, means an alkylcarbonyl group, as defined herein, appended to the parent molecular moiety through an oxygen atom. Representative examples of alkylcarbonyloxy include, but are not limited to, acetyloxy, ethylcarbonyloxy, and tert-butylcarbonyloxy.

The term "alkylthio" as used herein, means an alkyl group, as defined herein, appended to the parent molecular moiety through a sulfur atom. Representative examples of alkylthio include, but are not limited, methylthio, ethylthio, tert-butylthio, and hexylthio.

The term "alkylthioalkyl" as used herein, means an alkylthio group, as defined herein, appended to the parent molecular moiety through an alkyl group, as defined herein. Representative examples of alkylthioalkyl include, but are not limited, methylthiomethyl and 2-(ethylthio)ethyl.

The term "alkynyl" as used herein, means a straight or branched chain hydrocarbon group containing from 2 to 10 carbon atoms and containing at least one carbon-carbon triple bond. Representative examples of alkynyl include, but are not limited, to acetylenyl, 1-propynyl, 2-propynyl, 3-butynyl, 2-pentynyl, and 1-butynyl.

The term "aryl," as used herein, means a phenyl group or a naphthyl group.

The aryl groups of the present invention can be optionally substituted with one, two, three, four, or five substituents independently selected from the group consisting of alkenyl, alkoxy, alkoxyalkyl, alkoxycarbonyl, alkyl, alkylcarbonyl, alkylcarbonyloxy, alkylthio, alkylthioalkyl, alkynyl, carboxy, cyano, formyl, haloalkoxy, haloalkyl, halogen, hydroxy, hydroxyalkyl, mercapto, nitro, -NR_{E}R_{F}, and (NR_{E}R_{F})carbonyl

The term "arylalkyl" as used herein, means an aryl group, as defined herein, appended to the parent molecular moiety through an alkyl group, as defined herein. Representative examples of arylalkyl include, but are not limited to, benzyl, 2-phenylethyl, 3-phenylpropyl, 1-methyl-3-phenylpropyl, and 2-naphth-2-ylethyl.

The term "cancer," as used herein, means growth of tumor cells which interfere with the growth of healthy cells.

The term "carbonyl" as used herein, means a -C(O)- group.

The term "carboxy" as used herein, means a -CO₂H group.

The term CNS tumor, as used herein, means a tumor of the central nervous system (CNS), including brain stem glioma, craniopharyngioma, medulloblastoma, and meningioma.

The term "cyano" as used herein, means a -CN group.

The term "cycloalkyl" as used herein, means a saturated cyclic hydrocarbon group containing from 3 to 8 carbons, examples of cycloalkyl include cyclopropyl, cyclobutyl, cyclopentyl, cyclohexyl, cycloheptyl, and cyclooctyl.

The cycloalkyl groups of the present invention are optionally substituted with 1, 2, 3, or 4 substituents selected from alkenyl, alkoxy, alkoxyalkyl, alkoxycarbonyl, alkyl, alkylcarbonyl, alkylcarbonyloxy, alkylthio, alkylthioalkyl, alkynyl, carboxy, cyano, formyl, haloalkoxy, haloalkyl, halogen, hydroxy, hydroxyalkyl, mercapto, oxo, -NR_{E}R_{F}, and (NR_{E}R_{F}:)carbonyl.

The term "cycloalkylalkyl" as used herein, means a cycloalkyl group, as defined herein, appended to the parent molecular moiety through an alkyl group, as defined herein. Representative examples of cycloalkylalkyl include, but are not limited to, cyclopropylmethyl, 2-cyclobutylethyl, cyclopentylmethyl, cyclohexylmethyl, and 4-cycloheptylbutyl.

The term cytotoxic agent as used herein means a substance that is potentially genotoxic, oncogenic, mutagenic, teratogenic or in any way hazardous to cells; used commonly in referring to antineoplastic drugs that selectively damage or destroy dividing cells.

The term "formyl" as used herein, means a -C(O)H group.

The term "halo" or "halogen" as used herein, means -Cl, -Br, -I or -F.

The term "haloalkoxy" as used herein, means at least one halogen, as defined herein, appended to the parent molecular moiety through an alkoxy group, as defined herein. Representative examples of haloalkoxy include, but are not limited to, chloromethoxy, 2-fluoroethoxy, trifluoromethoxy, and pentafluoroethoxy.

The term "haloalkyl" as used herein, means at least one halogen, as defined herein, appended to the parent molecular moiety through an alkyl group, as defined herein. Representative examples of haloalkyl include, but are not limited to, chloromethyl, 2-fluoroethyl, trifluoromethyl, pentafluoroethyl, and 2-chloro-3-fluoropentyl.

The term "hoteroaryl," as used herein, means a monocyclic heteroaryl ring or a bicyclic heteroaryl ring. The monocyclic heteroaryl ring is a 5 or 6 membered ring. The 5 membered ring has two double bonds and contains one, two, three or four heteroatoms independently selected from the group consisting ofN, O, and S. The 6 membered ring has three double bonds and contains one, two, three or four heteroatoms independently selected from the group consisting ofN, O, and S. The bicyclic heteroaryl ring consists of the 5 or 6 membered heteroaryl ring fused to a phenyl group or the 5 or 6 membered heteroaryl ring is fused to another 5 or 6 membered heteroaryl ring. Nitrogen heteroatoms contained within the heteroaryl may be optionally oxidized to the N-oxide. The heteroaryl is connected to the parent molecular moiety through any carbon atom contained within the heteroaryl while maintaining proper valence. Representative examples of heteroaryl include, but are not limited to, benzothienyl, benzoxadiazolyl, cinnolinyl, furopyridinyl, furyl, imidazolyl, indazolyl, indolyl, isoxazolyl, isoquinolinyl, isothiazolyl, naphthyridinyl; oxadiazolyl, oxazolyl, pyridinyl, pyridazinyl, pyrimidinyl, pyrazinyl, pyrazolyl, pyrrolyl, pyridinium N-oxide, quinolinyl, tetrazolyl, thiadiazolyl, thiazolyl, thienopyridinyl, thienyl, triazolyl, and triazinyl.

The heteroaryl groups of the present invention are substituted with 0, 1, 2, 3, or 4 substituents independently selected from alkenyl, alkoxy, alkoxyalkyl, alkoxycarbonyl, alkyl, alkylcarbonyl, alkylcarbonyloxy, alkylthio, alkylthioalkyl, alkynyl, carboxy, cyano, formyl, haloalkoxy, haloalkyl, halogen, hydroxy, hydroxyalkyl, mercapto, nitro, -NR_{E}R_{F}, and (NR_{E}R_{F})carbonyl.

The term "heteroarylalkyl" as used herein, means a heteroaryl, as defined herein, appended to the parent molecular moiety through an alkyl group, as defined herein. Representative examples of heteroarylalkyl include, but are not limted to, pyridinymethyl.

The term "heterocycle" or "heterocyclic" as used herein, means a monocyclic or bicyclic heterocyclic ring. The monocyclic heterocyclic ring consists of a 3, 4, 5, 6, 7, or 8 membered ring containing at least one heteroatom independently selected from O, N, and S. The 3 or 4 membered ring contains 1 heteroatom selected from the group consisting of O, N and S. The 5 membered ring contains zero or one double bond and one, two or three heteroatoms selected from the group consisting of O, N and S. The 6 or 7 membered ring contains zero, one or two double bonds and one, two or three heteroatoms selected from the group consisting of O, N and S. The bicyclic heterocyclic ring consists of a monocyclic heterocyclic ring fused to a cycloalkyl group or the monocyclic heterocyclic ring fused to a phenyl group or the monocyclic heterocyclic ring fused to another monocyclic heterocyclic ring. The heterocycle is connected to the parent molecular moiety through any carbon or nitrogen atom contained within the heterocycle while maintaining proper valence. Representative examples of heterocycle include, but are not limited to, azetidinyl, azepanyl, aziridinyl, diazepanyl, 1,3-dioxanyl, 1,3-dioxolanyl, 1,3-dithiolanyl, 1,3-dithianyl, imidazolinyl, imidazolidinyl, isothiazolinyl, isothiazolidinyl, isoxazolinyl, isoxazolidinyl, morpholinyl, oxadiazolinyl, oxadiazolidinyl, oxazolinyl, oxazolidinyl, piperazinyl, piperidinyl, pyrazolinyl, pyrazolidinyl, pyrrolinyl, pyrrolidinyl, tetrahydrofuranyl, tetrahydropyranyl, tetrahydrothienyl, thiadiazolinyl, thiadiazolidinyl, thiazolinyl, thiazolidinyl, thiomorpholinyl, 1,1-dioxidothiomorpholinyl (thiomorpholine sulfone), thiopyranyl, and trithianyl.

The heterocycles of this invention are substituted with 0, 1, 2, or 3 substituents independently selected from alkenyl, alkoxy, alkoxyalkyl, alkoxycarbonyl, alkyl, alkylcarbonyl, alkylcarbonyloxy, alkylthio, alkylthioalkyl, alkynyl, carboxy, cyano, formyl, haloalkoxy, haloalkyl, halogen, hydroxy, hydroxyalkyl, mercapto, nitro, -NR_{E}R_{F}, and (NR_{E}RF)carbonyl.

The term "heterocycloalkyl" as used herein, means a heterocycle, as defined herein, appended to the parent molecular moiety through an alkyl group, as defined herein.

The term "hydroxy" as used herein, means an -OH group.

The term "hydroxyalkyl" as used herein, means at least one hydroxy group, as defined herein, is appended to the parent molecular moiety through an alkyl group, as defined herein. Representative examples of hydroxyalkyl include, but are not limited to, hydroxymethyl, 2-hydroxyethyl, 3-hydroxypropyl, 2,3-dihydroxypentyl, and 2-ethyl-4-hydroxyheptyl.

The term "mammal," as used herein, means a particular class of vertebrate.

The term "mercapto" as used herein, means a -SH group.

The term "nitro" as used herein, means a -NO₂ group.

The term "nonaromatic" as used herein, means that a 4 membered nonaromatic ring contains zero double bonds, a 5 membered nonaromatic ring contains zero or one double bond, a 6, 7, or 8 membered nonaromatic ring contains zero, one, or two double bonds.

The term "NR_{A}R_{B}" as used herein, means two groups, R_{A} and R_{B}, which are appended to the parent molecular moiety through a nitrogen atom. R_{A} and R_{B} are each independently hydrogen, alkyl, and alkylcarbonyl. Representative examples of NR_{A}R_{B} include, but are not limited to, amino, methylamino, acetylamino, and acetylmethylamino.

The term "(NR_{A}R_{B})carbonyl" as used herein, means a NR_{A}R_{B} group, as defined herein, appended to the parent molecular moiety through a carbonyl group, as defined herein. Representative examples of (NR_{A}R_{B})carbonyl include, but are not limited to, aminocarbonyl, (methylamino)carbonyl, (dimethylamino)carbonyl, and (ethylrnethylarnino)carbonyl.

The term "NR_{C}R_{D}" as used herein, means two groups, R_{C} and R_{D}, which are appended to the parent molecular moiety through a nitrogen atom. R_{C} and R_{D} are each independently, hydrogen, alkyl, and alkylcarbonyl. Representative examples of NR_{C}R_{D} include, but are not limited to, amino, methylamino, acetylamino, and acetylmethylamino.

The term "(NR_{C}R_{D})carbonyl" as used herein, means a NR_{C}R_{D} group, as defined herein, appended to the parent molecular moiety through a carbonyl group, as defined herein. Representative examples of (NR_{C}R_{D})carbonyl include, but are not limited to, aminocarbonyl, (methylamino)carbonyl, (dimethylamino)carbonyl, and (ethylmethylamino)carbonyl.

The term "(NR_{C}R_{D})carbonylalkyl" as used herein, means a (NR_{C}R_{D})carbonyl group, as defined herein, appended to the parent molecular moiety through an alkyl group, as defined herein.

The term "(NR_{C}R_{D})sulfonyl" as used herein, means a NR_{C}R_{D} group, as defined herein, appended to the parent molecular moiety through a sulfonyl group, as defined herein. Representative examples of (NR_{C}R_{D})sulfonyl include, but are not limited to, aminosulfonyl, (methylamino)sulfonyl, (dimethylamino)sulfonyl, and (ethylmethylamino)sulfonyl.

The term "NR_{E}R_{F}" as used herein, means two groups, R_{E} and R_{F}, which are appended to the parent molecular moiety through a nitrogen atom. R_{E} and R_{F} are each independently hydrogen, alkyl, and alkylcarbonyl. Representative examples of NR_{E}R_{F} include, but are not limited to, amino, methylamino, acetylamino, and acetylmethylamino.

The term "(NR_{E}R_{F})carbonyl" as used herein, means a NR_{E}R_{F} group, as defined herein, appended to the parent molecular moiety through a carbonyl group, as defined herein. Representative examples of (NR_{E}R_{F})carbonyl include, but are not limited to, aminocarbonyl, (methylamino)carbonyl, (dimethylamino)carbonyl, and (ethylmethylamino)carbonyl.

The term "oxo" as used herein, means a =O moiety.

The term radiotherapy as used herein, means exposure to radiation from a radioactive substance used in the treatment of disease (especially cancer).

The term or abbreviation, TMZ, as used herein means temozolomide.

The term "treating,'" as used herein, means at least sustaining and preferably reversing the course of a disease or adverse physiological event.

When used in the above or other treatments, a therapeutically effective amount of one of the compounds of the present invention can be employed as a zwitterion or as a pharmaceutically acceptable salt. By a "therapeutically effective amount" of the compound of the invention is meant a sufficient amount of the compound to treat or prevent a disease or disorder ameliorated by a PARP inhibitor at a reasonable benefit/risk ratio applicable to any medical treatment. It will be understood, however, that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidential with the specific compound employed; and like factors well known in the medical arts. For example, it is well within the skill of the art to start doses of the compound at levels lower than those required to achieve the desired therapeutic effect and to gradually increase the dosage until the desired effect is achieved.

By "pharmaceutically acceptable salt" is meant those salts which are, within the scope of sound medical judgement, suitable for use in contact with the tissues of humans and lower animals without undue toxicity, irritation, allergic response and the like and are commensurate with a reasonable benefit/risk ratio. Pharmaceutically acceptable salts are well-known in the art. The salts can be prepared in situ during the final isolation and purification of the compounds of the present invention or separately by reacting the free base of a compound of the present invention with a suitable acid. Representative acids include, but are not limited to acetatic, citric, aspartic, benzoic, benzenesulfonic, butyric, fumaric, hydrochloric, hydrobromic, hydroiodic, lactic, maleic, methanesulfonic; pamoic, pectinic, pivalic, propionic, succinic, tartaric, phosphic, glutamic, and p-toluenesulfonic. Also, the basic nitrogen-containing groups can be quatemized with such agents as lower alkyl halides such as methyl, ethyl, propyl, and butyl chlorides, bromides and iodides; dialkyl sulfates like dimethyl, diethyl, dibutyl and diamyl sulfates; long chain halides such as decyl, lauryl, myristyl and stearyl chlorides, bromides and iodides; arylalkyl halides like benzyl and phenethyl bromides and others. Water or oil-soluble or dispersible products are thereby obtained.

A compound of the present invention may be administered as a pharmaceutical composition containing a compound of the present invention in combination with one or more pharmaceutically acceptable excipients. A pharmaceutically acceptable carrier or excipient refers to a non-toxic solid, semi-solid or liquid filler, diluent, encapsulating material or formulation auxiliary of any type. The compositions can be administered parenterally, intracisternally, intravaginally, intraperitoneally, topically (as by powders, ointments, drops or transdermal patch), rectally, or bucally. The term "parenteral" as used herein refers to modes of administration which include intravenous, intramuscular, intraperitoneal, intrasternal, subcutaneous and intraarticular injection and infusion.

Pharmaceutical compositions for parenteral injection comprise pharmaceutically-acceptable sterile aqueous or nonaqueous solutions, dispersions, suspensions or emulsions, as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol, and the like), carboxymethylcellulose and suitable mixtures thereof, vegetable oils (such as olive oil), and injectable organic esters such as ethyl oleate. Proper fluidity may be maintained, for example, by the use of coating materials such as lecithin, by the maintenance of the required particle size in the case of dispersions, and by the use of surfactants.

These compositions can also contain adjuvants such as preservative, wetting agents, emulsifying agents, and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents such as sugars, sodium chloride, and the like. Prolonged absorption of the injectable pharmaceutical form may be brought about by the inclusion of agents which delay absorption, such as aluminum monostearate and gelatin.

Compounds of the present invention may also be administered in the form of liposomes. As is known in the art, liposomes are generally derived from phospholipids or other lipid substances. Liposomes are formed by mono- or multi-lamellar hydrated liquid crystals that are dispersed in an aqueous medium. Any non-toxic, physiologically-acceptable and metabolizable lipid capable of forming liposomes can be used. The present compositions in liposome form can contain, in addition to a compound of the present invention, stabilizers, preservatives, excipients, and the like. The preferred lipids are the phospholipids and the phosphatidyl cholines (lecithins), both natural and synthetic. Methods to form liposomes are known in the art. See, for example, Prescott, Ed., Methods in Cell Biology, Volume XIV, Academic Press, New York, N.Y. (1976), p. 33 *et seq.*

Total daily dose of the compositions of the invention to be administered to a human or other mammal host in single or divided doses may be in amounts, for example, from 0.0001 to 300 mg/kg body weight daily and more usually 1 to 300 mg/kg body weight. The dose, from 0.0001 to 300 mg/kg body, may be given twice a day.

Compounds of the present invention were named by ACD/ChemSketch version 5.06 (developed by Advanced Chemistry Development, Inc., Toronto, ON, Canada) or were given names which appeared to be consistent with ACD nomenclature.

### Determination of Biological Activity

### Inhibition of PARP

Nicotinamide[2,5',8-3H]adenine dinucleotide and strepavidin SPA beads were purchased from Amersham Biosiences (UK) Recombinant Human Poly(ADP-Ribose) Polymerase (PARP) purified from E.coli and 6-Biotin-17-NAD⁺ , were purchase from Trevigen, Gaithersburg, MD. NAD⁺, Histone, aminobenzamide, 3-amino benzamide and Calf Thymus DNA (dcDNA) were purchased from Sigma, St. Louis, MO. Stem loop oligonucleotide containing MCAT sequence was obtained from Qiagen. The oligos were dissoloved to 1mM in annealing buffer containing 10mM Tris HCl pH 7.5, 1mM EDTA, and 50mM NaCl, incubated for 5min at 95°C, and followed by annealing at 45°C for 45 minutes. Histone Hl (95% electrophoretically pure) was purchased from Roche, Indianapolis, IN. Biotinylated histone Hl was prepared by treating the protein with Sulfo-NHS-LC-Biotin from Pierce Rockford, IL. The biotinylation reaction was conducted by slowly and intermittently adding 3 equivalents of 10mM Sulfo-NHS-LC-Biotin to 100µM Histone H1 in phosphate-buffered saline, pH 7.5, at 4°C with gentle vortexing over 1min followed by subsequent 4°C incubation for 1hr. Streptavidin coated (FlashPlate Plus) microplates were purchased from Perkin Elmer, Boston, MA.

PARP 1 assay was conducted in PARP assay buffer containing 50 mM Tris pH 8.0, 1mM DTT, 4 mM MgCl₂. PARP reactions contained 1.5 µM [³H]-NAD⁺(1.6uCi/mmol), 200 nM biotinylated histone H1, 200 nM slDNA, and 1nM PARP enzyme. Auto reactions utilizing SPA bead-based detection were carried out in 100µl volumes in white 96 well plates. Reactions were initiated by adding 50 µl of 2X NAD⁺ substrate mixture to 50µl of 2X enzyme mixture containing PARP and DNA. These reactions were terminated by the addition of 1.50 µl of 1.5 mM benzamide (~1000-fold over its IC50). 170 µl of the stopped reaction mixtures were transferred to streptavidin Flash Plates, incubated for 1hr, and counted using a TopCount microplate scintillation counter. The Kᵢ data was determined from inhibition curves at various substrate concentrations and are shown in Table 1 for representative compounds of the present invention.

**Table 1**

| Inhibition of PARP | |
|---|---|
| | PARP Inhibition |
| Compound | Kᵢ (nM) |
| 2-(2-methylpyrrolidin-2-yl)-1H-benzimidazole-4-carboxamide * | 4.3 |
| 2-[(2R)-pyrrolidin-2-yl]-1H-benzimidazole-4-carboxamide * | 8 |
| 2-[(2R)-2-mothylpyrrolidin-2-yl]-1H-benzimidazole-4-carboxamide | 5.4 |
| 2-[(2S),pyrrolidin-2-yl]-1H-benzimidazole-4-caxboxamide-4 *- | 28.4 |
| 2-[(2S)-2-methylpyrrolidin-2-yl]-1H-benzimidazole-4-carboxamide * | 5.1 |
| 2-[(2S)-1-methylpyrrolidin-2-yl]-1H-benzimidazole-4-carboxamide * | 30.8 |
| 2-[(2R)-1-methylpyrrolidin-2-yl]-1H-benzimidazole-4-carboxamide * | 7.3 |
| 2-(1,2-dimethylpyrrolidin-2-yl)-1H-benzimidazole-4-carboxamide * | 6.2 |
| 2-[(2S)-1-ethylpyrrolidin-2-yl]-1H-benzimidazal-4-carboxamide * | 49 |
| 2-(1-ethyl-2-methylpyrrolidin-2-yl)-1H-bonzimidazole-4-carboxamide * | 6 |
| 2-[(2S)-1-propylpyrrolidin-2-yl]-1H-benzimidazolez-4-carboxamide * | 129 |
| 2-[(2R)-1-propylpyrrolidin-2-yl]-1H-benztmidazole-4-carboxamide * | 146 |
| 2-(2-methyl-1-propylpyrrolidin-2-yl)-1H-benzimidazole-4-carboxamide * | 18.7 |
| 2-[(2R)-1-isopropylpyrrolidin-2-yl]-1H-benzimidazole-4-carboxamide * | 12.8 |
| 2-[(2S)-1-isopropylpyrrolidin-2-yl]-1H-benzimidazole-4-carboxamide * | 19.3 |
| 2-(1-isopropyl-2-mothylpyrrolidin-2-yl)-1H-benzimidazole-4-carboxamide * | 17.5 |
| 2-[(2S).-1-cyelobutylpyrrolidin-2-yl]-1H-benzimidazole-4-carboxamide * | 338 |
| 2-[(2R)-1-cyclobutypyrrolidin-2-yl]-1H-benzimidazole-4-carboxamide * | 142 |
| 2-(1-cyclobutyl-2-methylpyrrolidin-2-yl)-1H-benzimidazole-4-carboxamide * | 31.3 |
| 2-pyrrolidin-3-yl-1H-benzimidazole-4-carboxamide * | 3.9 |
| 2-(3-methylpyrrolidin-3-yl)-1H-benzimidazole-4-carboxamide * | 3.9 |
| 2-(1-propylpyrrolidin-3-yl)-1H-benzimidazole-4-carboxamide * | 8.1 |
| 2-(3-methyl-l-propylpyrrolidin-3-yl)-1H-benzimidazole-4-carbaxamide * | 4.2 |
| 2-[1-(cyclopropylmethyl)pyrrolidin-3-yl]-1H-benzimidazole-4-carboxamide * | 5.2 |
| 2-[1-(cyclopropylmethyl)-3-methylpyrrolidin-3-yl]-1H-benzimidazole-4-carboxamide * | 5 |
| 2-(1-isobutylpyrrolidin-3-yl)-1H-benzimidazole-4-carboxamide * | 7.4 |
| 2-(1-isobutyl-3-methylpyrrolidin-3-yl)-1H-benzimidazole.-4-carboxamide * | 3.8 |
| 2-(1-isopropylpyrrolidin-3-yl)-1H-benzimidazole-4-carboxamide | 9.2 |
| 2-(1-isopropyl-3-methylpyrrolidul-3-yl)-1H-benzimidazole-4-carboxamide * | 4.4 |
| 2-(1-cyclobutylpyrrolidin-3-yl)-1H-benzimidazole-4-carboxamide * | 6.8 |
| 2-(1-cydobutyl-3-methylpyuolidin-3-yl)-1H benzimidazole-4-carboxamide * | 4 |
| 2-(1-cyclopentylpyrrolidin-3-yl)-1H-benzimidazole-4-carboxamide * | 5.5 |
| 2-(1-cylopentyl-3-methylpyrrolidin-3-yl)-1H-benzimidazole-4-carboxamide * | 3.4 |
| 2-(1-cyclohexylpyrrolidin-3-yl)-1H-benzimidazole-4-carboxamide * | 7 |
| 2-(1-coyclohexyl-3-methylpyrrolidin-3-yl)-1H-benzimidazole-4-carboxamide * | 5,8 |
| 2-(1-tetrahydro-2H-pyran-4-ylpyrrolidin-3-yl)-1H-benzimidazole-4-carboxamide * | 8,2 |
| 2-(3-methyl-1-tetrahydro-2H-pyran-4-ylpyrrolidin-3-yl)-1H-benzimidazole-4-carboxamide * | 7.2 |
| 2-[1-(pyridin-4-ylmethyl)pyrrolidin-3-yl]-1H-benzimtdazole-4-carboxamide * | 14.2 |
| 2-[3-methyl-1-(pyridin-4-ylmethyl)pyrrolidin-3-yl]-1H-benzimidazole-4-carboxamide * | 8.9 |
| 2-[1-(2-phenylethyl)pyrrolidin-3-yl]-1H-benzimidazole-4-carboxamide * | 9.1 |
| 2-[3-methyl-1-(2-phenylethyl)pyrrolidin-3-yl]-1H-benzimidazole-4-carboxamide * | 10,5 |
| 2-[1-(1-methyl-3-phenylpropyl)pyrrolidin-3-yl]-1H-benzimidazole-4-Carboxamide * | 13:2 |
| 2-[3-methyl-1-(1-methyl-3-phenylpropyl)pyrrolidin-3-yl]-1H-benzimidazole-4-carboxamide * | 12 |
| 2-azetidin-2-yl-1H-benzimidazole-4-carboxamide * | 34 |
| 2-(2-methylazetidin-2-yl)-1H-benzimidazole-4-carboxamide * | 14.1 |
| 2-(1-isopropylazetidin-2-yl)-1H-benzimidazole-4-caxboxamide * | 118 |
| 2-(1-isopropyl-2-methylazetidin-2-yl)-1H-benzimidazole-4-carboxamide * | 41.6 |
| 2-(1-cyclobutylazetidin-2-yl)-1H-benzimidazole-4-carboxamide * | 80 |
| 2-(1-cyclobutyl-2-methylazetidin-2-yl)-1H-benzimidazole-4-carboxamide * | 33.3 |
| 2-(1-cyclopentylazetidin-2-yl)-1H-benzimidazole-4-carboxamide * | 176 |
| 2-(1-cyclopentyl-2-methylazetidin-2-yl)-1H-benzimidazole-4-carboxamide * | 31.1 |
| 2-(1-cyclohexylazetidin-2-yl)-1H-benzimidazole-4-carboxamide * | 245 |
| 2-(1-eyelohoxyl-2-methylazetidin-2-yl)-1H-benzimidazole-4-carboxamide * | 27.7 |
| 2-azetidin-3-yl-1H-benzimidazole-4-carboxamide * | 6 |
| 2-(3-methylazettdin-3-yl)-1H-benzimidazole-4-carboxamide * | 4.4 |
| 2-(1-propylazetidin-3-yl)-1H-benzimidazole-4-carboxamide * | 14.1 |
| 2-(3-methyl-l-propylazetidin-3-yl)1H-benzimidazole-4-carboxamide * | 6.9 |
| 2-[1-(cylopropylmethyl)azetidin-3-yl]-1H-benzimidazole-4-carboxamide * | 19 |
| 2-[1-(cyclopropylmethyl)-3-methylazetidin-3-yl]-1H-benzimidazole-4-carboxamide * | 8 |
| 2-(1-isobutylazetidin-3-yl)-1H-benzimidazole-4-carboxamide * | 14.4 |
| 2-(1-isobutyl-3-methylazetidin-3-yl)-1H-benzimidazole-4-carboxamide * | 5.6 |
| 2-(1-cyclobutylazetidin-3-yl)-1H-benzimidazole-4-carboxamide * | 16.4 |
| 2-(1-cyclobutyl-3-methylazetidin-3-yl)-1H-benzimidazole-4-carboxamide * | 6.1 |
| 2-(1-cyclopentylazetidin-3-yl)-1H-benzimidazole-4-carboxamide * | 14 |
| 2-(1-cyclopentyl-3-methylazetidin-3-yl)-1H-benzimidazole-4-carboxamide * | 4 |
| 2-(1-cyclohexylazetidin-3-yl)-1H-benzimidazole-4-carboxamide * | 16 |
| 2-(1-cyclohexyl-3-methylazetidin-3-yl)-1H-benzimidazole-4-carboxamide * | 5.6 |
| 2-(1-tetrahydro-2H-pyran-4-ylazetidin-3-yl)-1H-benzimidazole-4-carboxamide * | 45.6 |
| 2-(3-methyl-1-tetrahydro-2H-pyran-4-ylazetidin-3-yl)-1H-benzimidazole-4-carboxamide * | 12.7 |
| 2-{1-[(dimethylamino)sulfonyl]azetidin-3-yl}-1H-benzimidazole-4-calboxamide * | 16 |
| 2-{1-[(dtmethylamino)sulfonyl]-3-methylazetidin-3-yl}-1H-benzimidazole-4-carboxamide * | 7 |
| 2-[(2S)-piperidin-2-yl]-1H-benzimidazole-4-carboxamide * | 46.1 |
| 2-[(2R) -piperidin-2-yl]-1H-benziraidazole-4-carboxamide * | 47.4 |
| 2-[piperidin-2-y1]-1H-benzimidazole-4-carboxamide * | 32.2 |
| 2-(2-methylpiperidin-2-yl)-1H-benzimidazole-4-carboxamide * | 4.6 |
| 2-(1-propylpiperidin-2-yl)-1H-benzimidazole-4-carboxamide * | 120 |
| 2-(2-methyl-l-propylpiperidin-2-yl)-1H-benzimidazole-4-carboxamide * | 18.7 |
| 2- {1-[(dimethylamino)sulfonyl]piperidin-4-yl) -1H-benzimidazole-4-carboxamide * | 31.1 |
| 2- {1-[(dimethylamino)sulfonyl]-4-methylpiperidin-4-yl}-1H-benzimidazole-4-carboxamide * | 8.8 |
| 2-(1-cyclobutylpiperidin-4-yl)-1H-benzimidazole-4-carboxamide * | 6.3 |
| 2-(1-cyclobutyl-4-methylpipendin-4-yl)-1H-benzimidazole-4-carboxamide * | 9.2 |
| 2-(1-isopropylpiperidin-4-y1)-1H-benzimidazole-4-carboxamide * | 6 |
| 2-(1-isopropyl-4-methylpiperidin-4-yl)-1H-benzimidazole-4-carboxamide * | 8 |
| 2-(N-propylpiperidin-4-yl) benzimidazole-4-carboxamide * | 8.6 |
| 2-(4-methyl-1-propylpiperidin-4-yl)-1H-benzimidazole-4-carboxamide * | 13.5 |
| 2-azepan-4-yl-1H-benzimidazole-4-carboxamide * | 5.7 |
| 2-(4-methylazepan-4-yl)-1H-benzimidazole-4-carboxamide * | 3.3 |
| 2-(1-cyclopentylazepan-4-yl)-1H-benzimidazole-4-carboxamide * | 3.9 |
| 2-(1-cyclopentyl-4-methylazepan-4-yl)-1H-benzimidazole-4-carboxamide * | 7.3 |
| 2-(1-cyclohexylazepan-4-yl)-1H-benzimidazole-4-carboxamide * | 4.8 |
| 2-(1-cyclohexyl-4-methylazepan-4-yl)-1H-benzimidazole-4-carboxamide * | 11.9 |

| | |
|---|---|
| * not according to the present invention (comparative data). | |

The following examples are presented to provide what is believed to be the most useful and readily understood description of procedures and conceptual aspects of this invention.

### In Vivo Assay (not according to the present invention)

This study was done in nude mice bearing HCT-116 tumors in the leg. Three days (-3) prior to the beginning of radiotherapy, mice were implanted i.p with OMPs delivering A-620223 at 0,6.25,12.5, or 25 mg/kg/day for 14 days. Starting day 0 mice received radiation treatment (2 Gy/day) for 10 doses alone or in combination with the 3 different doses of 2-(N-propylpiperidin-4-yl) benzimidazole-4-carboxamide.

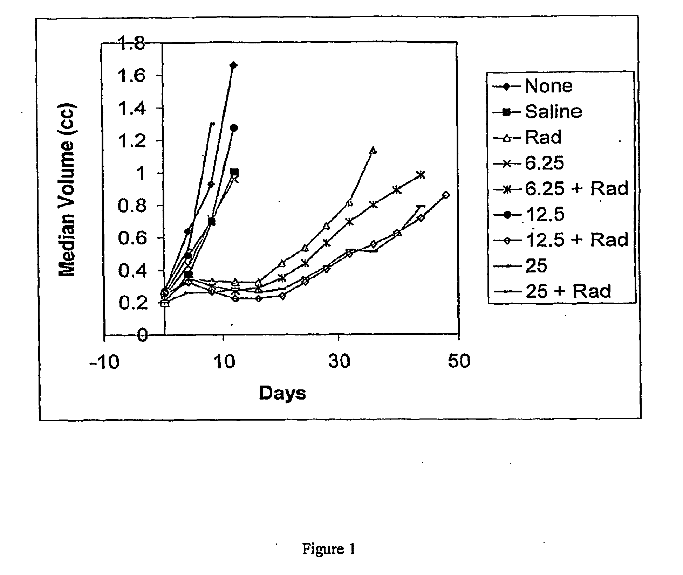

As can be seen from the data presented in Figure 1, the combination of the compound, 2-(N-propylpiperidin-4-yl) benzimidazole-4-carboxamide, with radiotherapy resulted in a significant improvement in the reduction of tumor size when compared to the administration of radiotherapy or compound alone as a monotherapy.

### In vivo Assay (not according to the present invention)

This study was done on mice with B16F10 murine melanoma. Mice were divided into six treatment groups with 8-10 mice per group. See figure two for treatment groups. B16F10 cells were injected s.c. into C57BL/6 mice on day 0. Dosing was initiated on day one. A-861695 was administered p.o., b.i.d. on days 1-14. On days 3-7 temozolomide (TMZ) was administered p.o., q.d. (for the groups receiving both TMZ and A-861695, TMZ was given two hours after the A-861695 was administered).

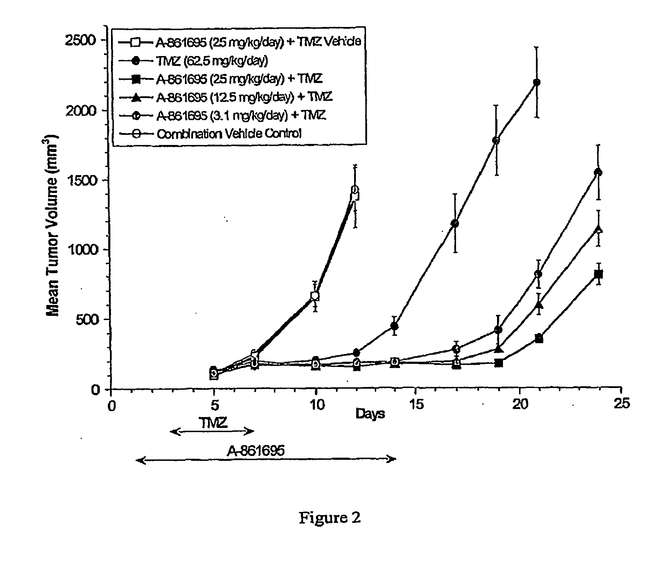

As can be seen from the data presented in Figure 2, A-861695, administered orally, significantly potentates the TMZ efficacy in a dose dependent manner. The combination of A-861695 at 25, 12.5 or 3.1 mg/kg/day p.o., divided b.i.d., in combination with TMZ at 62.5 mg/kg/day (p.o., q.d. X5) proved significantly more efficacious than TMZ monotherapy.

### In vivo Assay

This study was conducted with Fisher 344 rats. 9L is a transplantable rat glioma cell line that produces orthotopic gliosarcoma in Fisher 344 rats. Since 9L is implanted orthotopically, this model can be used to assess the ability of a compound to be effective in an environment where drug must cross the blood-brain barrier. Agents such as TMZ, which cross the blood-brain barrier, are more efficacious in this model than agents that do not.

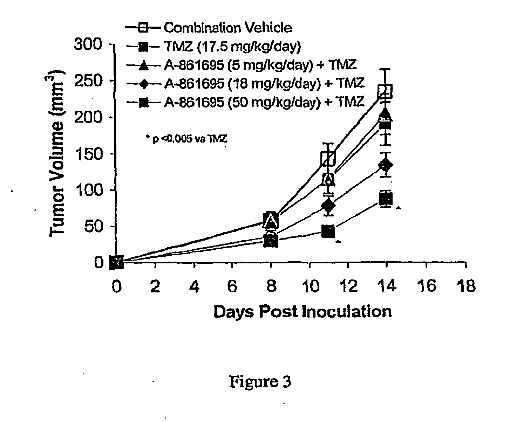

Rats were randomized into treatment groups (11-12 rats per group) of vehicle, TMZ (17.5 mg/kg/day, p.o. q.d.), and A-861695 (5, 18, and 50 mg/kg/day, p.o. b.i.d.) + TMZ (17.5 mg/kg, p.o. q.d.). Treatment of A-861695 began on day 3 following tumor cell inoculation and continued for 13 days. TMZ was administered from day 4 to 8. Tumor growth was monitored longitudinally using contrast-enhanced magnetic resonance imaging (MRI). Animal survival was evaluated based on humane euthanasia of rats presenting signs of irreversible illness. Results are shown in Figure 3.

When combined with TMZ, A-861695 significantly potentiated its antitumor activity. A-861695 at 50 mg/kg/day in combination with TMZ reduced tumor volume (on day 14) by 63%, which was 44% better than TMZ alone (p < 0.005). The combination of 18 mg/kg/day or 50 mg/kg/day doses of A-861695 with TMZ also significantly prolonged animal survival (p < 0.005, Log-rank test).

The pharmacokinetic profile of A-861695 was evaluated in tumor-bearing rats with drug concentration measured in plasma as well as in brain and tumor tissues. After multiple doses of A-861695 (50mg/kg/day), the concentration of the compound 2 hours post dosing (near Cₘₐₓ) was 1.36 ± 0:16 µg/mL, 0.72 ± 0.12 µg/g, and 3.00 ± 0.16 µg/g, in plasma, brain, and tumor tissues, respectively. A-861695 displayed improved bioavailability in brain tissue compare to other PARP inhibitors. Co-administration of TMZ did not alter the plasma PK profile of A-861695.

### In vivo Assay not according to the present invention

The MX-1 breast carcinoma xenograft model in scid mice was used to test the ability of A-861695 to potentiate the efficacy of platinum-based agents. This cell line was derived from a 29-year old female with a poorly differentiated mammary carcinoma. Mix-1 is sensitive to cytotoxic agents.

Carboplatin, a second-generation platinum containing anticancer drug, is currently the standard of care for treating lung, ovarian, and head and neck cancers. MX-1 tumors are sensitive to carboplatin. Therefore, carboplatin was administered at lower doses of 5,10, and 15 mg/kg/day to obtain an appropriate experimental window to allow examination of potentiation with PARP inhibitors.

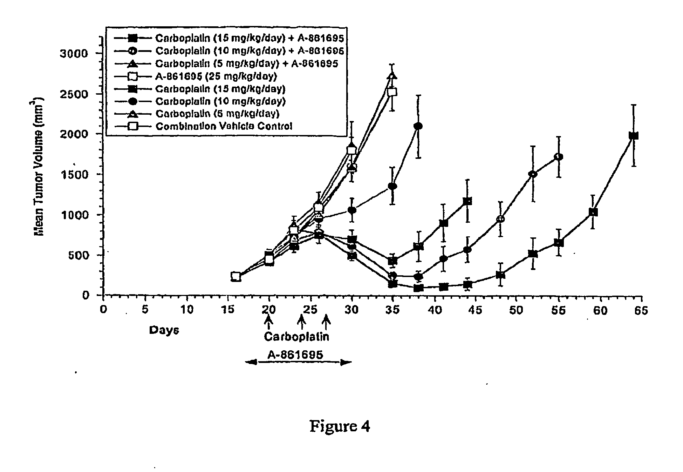

Mice were randomized into treatment groups of 8-10 mice per group. Tumors were size-matched to ~200 mm³ on day 16. A-861695 was administered at 25 mg/kg/day s.c., *via* 14-day osmotic minipumps (OMPs) starting on day 17. Carboplatin was given i.p., on day 20, 24 and 27. Data presented in Figure 4 are mean ± S.E.M. of 8-10 mice per treatment group.

As a single agent, carboplatin produced a dose-dependent tumor inhibition. A-861695 administered at 25 mg/kg/day *via* OMPs for 14 days caused a pronounced potentiation of carboplatin at 10 and 15 mg/kg/day as reflected by tumor volumes. The 10 mg/kg/day carboplatin/PARP combination regressed tumor volumes from day 26, whereas carboplatin monotherapy only delayed tumor growth.

### In vivo Assay not according to the present invention

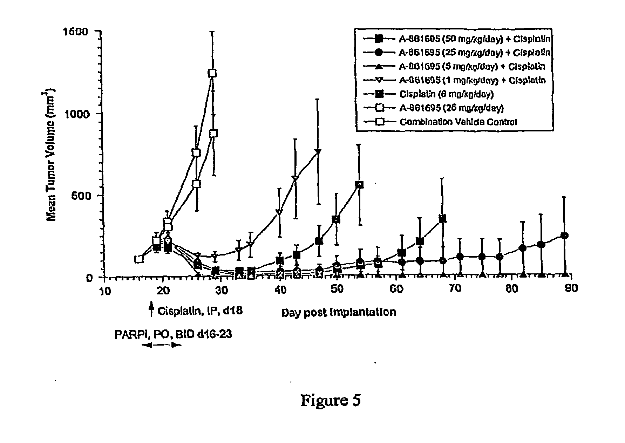

In this study the efficacy of A-861695 in combination with cisplatin was evaluated in the MX-1 breast carcinoma xenograft model in nude mice. Tumors were size-matched to 100 mm³ on day 16 and PARP inhibitor therapy (p.o., b.i.d. x8) was initiated the same day. A single dose of cisplatin at 6.0 mg/kg/day was administered i.p. day 18. Data, shown in Figure 5, are mean ± S.E.M. of 9 mice per treatment group.

A-861695 induced a pronounced potentiation of cisplatin activity. A-861695 at 5, 25, and 50 mg/kg/day in combination with cisplatin showed an increase in cures (8/9, 8/9 and 6/9 animals, respectively, cures defined as no measurable tumors at end of the trial), whereas the cisplatin monotherapy had only 3/9 cures. This dose-response study demonstrated that maximal potentiation was reached at 5 mg/kg/day of A-861695.

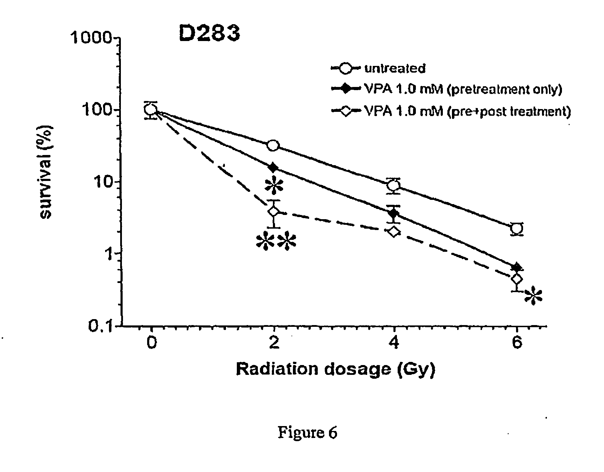

Applicants have also found HDAC inhibitors such as valproic acid can be used to reduce tumor size, Valproic acid crosses the blood brain barrier and is well studies and is safely tolerated in children, Valproic acid as a single therapeutic agent has been used as an anti-tumor agent for adult and pediatric tumors, including neuroblastomas and gliomas. Applicants have found that valproic acid can enhance the effects of radiotherapy (see figure 6). The parp inhibitor A-861695 also crosses the blood brain barrier and may work well in combination with valproic acid.

### DOSING

The dosing of compounds of form (I) such as 2-[(2R)-2-methylpyrrolidin-2-yl]-1H-benzimidazole-4-carboxamide in humans has been studied by Applicants. The following schedule, shown in table 2, has been used by Applicants when administering ABT-888 and temozolomide. This protocol for dosing can be followed for up to 12 cycles.

**Table 2**

| | DAY | | | | | | | | |
|---|---|---|---|---|---|---|---|---|---|
| DRUG | 1 | 2 | 3 | 4 | 5 | 6 | 7 | 8 | 9-28 |
| temozolomide | | X | X | X | X | X | | | Rest and Evaluation |
| ABT-888 | X | X | X | X | X | X | X | X | |

The following dose escalation schema, shown in Table 3, was used by Applicants to dose temozolomide. All patients were started with dose level 1. Patients with leukemia were dosed one level below the dose level under the study for patients with solid/CNS tumors. Table 4 shows the dose adjustment of temozolomide for patients with solid/CNS tumors. Table 5 shows the dose adjustment of temozolomide for patients with leukemias.

**Table 3**

| Temozolomide dose escalation schema | |
|---|---|
| Dose level | Dose |
| 0 | 125 mg/m²/day |
| 1 | 150 mg/m²/day |
| 2 | 175 mg/m²/day |
| 3 | 200 mg/m²/day |

**Table 4**

| **Day 29 ANC and/or Platelet Count** | | **Recovery** | **Dose Adjustment** |
|---|---|---|---|
| 500≤ ANC < 1000/µl | 50.000 ≤ Plt < 100,000/µl | Before day 42 from start of prior cycle | Resume TMZ without dose adjustment |
| 500 ≤ ANC < 1000/µl | 50,000 ≤Plt < 1 100,000/µl | After day 42 from start of prior cycle | Reduce TMZ dose by 25 mg/m²/day |
| ANC < 500 | Plt < 50,000/ml | N.A. | Reduce TMZ dose by 25 mg/m²/day |

**Table 5**

| |
|---|
| Protocol therapy to continue if ANC ≥ 500/µl and platelet count ≥ 20,000/µl by day 28 |
| If ANC ≥ 500/µl and platelet count ≥ 20.000/µl) by day 42 -> reduce TMZ by 25 mg/m2/day |
| If ANC ≤ 500/µl and/or platelet count ≤ 20,000/µl by day 42 -> bone marrow < 25% blasts |
| Postpone therapy until ANC ≥ 500/µl and platelet count ≥ 20,000/µl |
| Reduce TMZ by 25 mg/m2/day |

### Additional In Vivo Studies

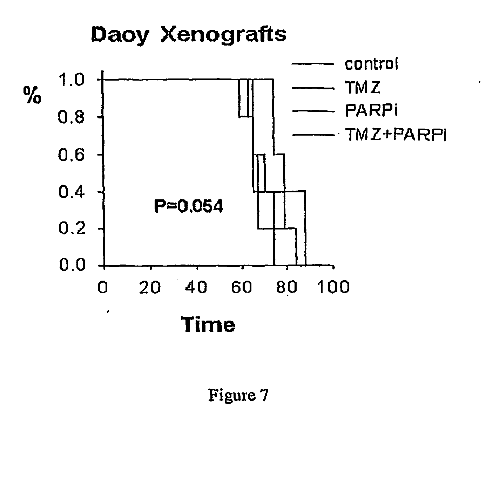

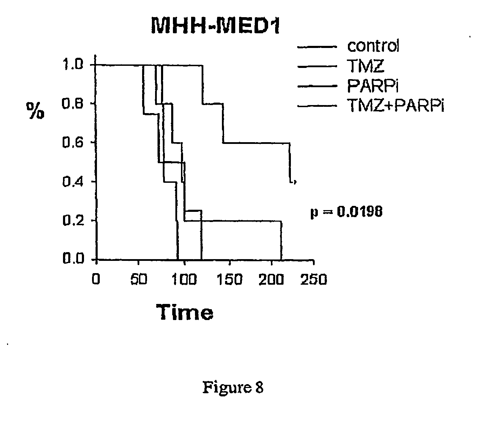

Percentage survival rate of mice with intra-cerebellar medulloblastoma xenographs after having been treated with TMZ and ABT-888 are shown in Figure 7 and 8. Time is in days.

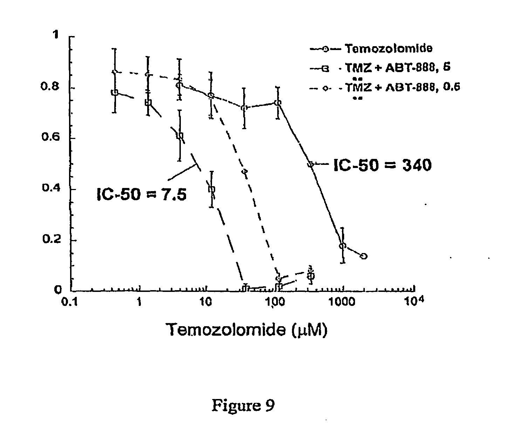

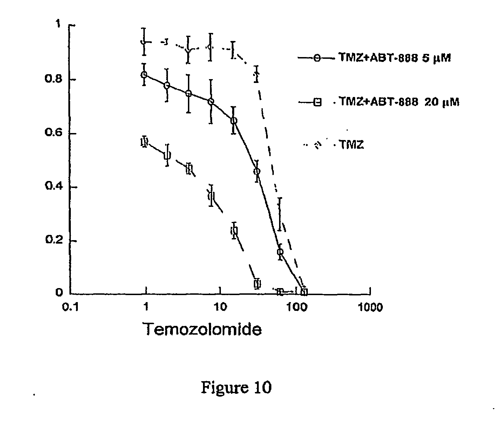

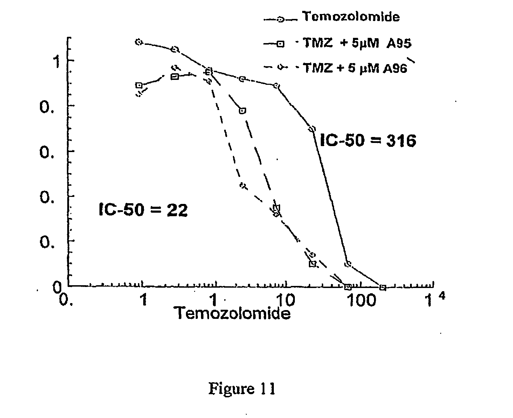

Results of administration and enhancement of in vivo activity of differing amounts of TMZ and ABT-888 combinations for HSB T-cell ALL; JM1 pre-B ALL; and P115 primary AML cells; are shown in Figures 9-11.

These data show the enhancement of toxicity of TMZ by ABT-888.

## Claims

1. A PARP inhibitor or a therapeutically acceptable salt thereof, wherein the PARP inhibitor is 2-[(2R)-2-methylpyrrolidin-2-yl]-1H-benzimidazole-4-carboxamide, for use in treating cancer or inhibiting tumor growth in a mammal, wherein the cancer is a CNS tumor, and wherein said PARP inhibitor is to be administered in combination with radiotherapy.

## Patentansprüche

1. Ein PARP-Inhibitor oder ein therapeutisch verträgliches Salz davon, worin der PARP-Inhibitor 2-[(2R)-2-Methylpyrrolidin-2-yl]-1H-benzimidazol-4-carboxamid ist, für die Verwendung in der Behandlung von Krebs oder in der Hemmung des Tumorwachstums in einem Säugetier, worin der Krebs ein ZNS-Tumor ist, und worin der PARP-Inhibitor in Kombination mit einer Strahlentherapie verabreicht werden soll.

## Revendications

1. Inhibiteur de PARP ou sel thérapeutiquement acceptable de celui-ci, où l'inhibiteur de PARP est le 2-[(2R)-2-méthylpyrrolidin-2-yl]-1H-benzimidazole-4-carboxamide, destiné à être utilisé dans le traitement du cancer ou l'inhibition de la croissance tumorale chez un mammifère, où le cancer est une tumeur du SNC, et où ledit inhibiteur de PARP est destiné à être administré en combinaison avec une radiothérapie.
